# EUROPEAN PATENT SPECIFICATION

(11) **EP 1 153 599 B1**
(45) Date of publication and mention of the grant of the patent: **05.03.2008**
(21) Application number: 01111103.6
(22) Date of filing: 08.05.2001
(51) Int. Cl.: A61Q 5/10, A61K 8/49

(54) **Hair dye composition**
Haarfärbemittel
Composition pour la teinture des cheveux

(30) Priority: 11.05.2000 JP 2000138466
(43) Date of publication of application: 14.11.2001
(73) Proprietor: KAO CORPORATION, Tokyo 103-8210 (JP)
(72) Inventor: Miyabe, Hajime, Kao Corporation, Sumida-ku, Tokyo 131-8501 (JP); Matsunaga, Kenichi, Kao Corporation, Sumida-ku, Tokyo 131-8501 (JP)
(74) Representative: HOFFMANN EITLE

(56) References cited:
- EP-A- 1 025 834
- WO-A-95/01772
- WO-A-99/20234
- US-A- 5 688 291

## Description

### Technical Field

The present invention relates to a hair dye composition which is, even at a basic pH, free of color loss during storage and can therefore keep its dyeing power, is capable of dyeing the hair in a deep, natural, brown to black color tone and has excellent color fastness to light, to shampooing and to friction.

### Background Art

In Japanese Language Laid-Open Publication (PCT) No. Hei 8-501322, disclosed is a cationic direct dye capable of dyeing the hair in a deep color and having excellent color fastness to light, to shampoo and to friction.

The higher the pH of the aqueous solution of the above-described cationic direct dye, the higher the hair dyeing property. It has however been proved that among the dyes as described in Japanese Language Laid-Open Publication (PCT) No. Hei 8-501322, blue dyes represented by the formula (1) (wherein X = S or O) or the formula (6) are accompanied with the drawback that when they are used as a basic solution, they gradually lose their color during storage and their dyeing power inevitably lowers. The blue color is indispensable for dyeing the hair with a natural brown or black color. Accordingly, the technique disclosed in the above patent gazette was not suited for dyeing the hair in a brown or black color at a high basic pH which otherwise enabled high dyeing power.

### Disclosure of the Invention

An object of the present invention is therefore to provide a hair dye which has excellent storage stability at a basic pH, is capable of dyeing the hair in a natural, deep, brown to black color tone, and has excellent color fastness.

The present inventors have found that a hair dye composition which can satisfy the above-described requirement is available by using in combination a cationic direct dye described in Japanese Language Laid-Open Publication (PCT) No. Hei 8-501322 and having a yellow to red color tone, and a specific cationic direct dye having a blue to purple color tone.

In the present invention, there is thus provided a hair dye composition which has a pH of 8 to 12 comprising (A) a first direct dye represented by any one of the following formulas (1) to 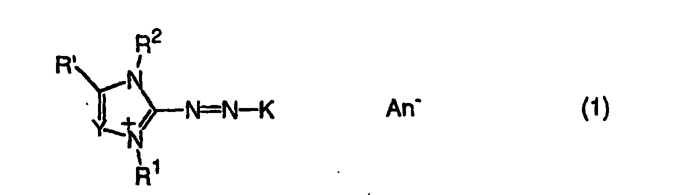 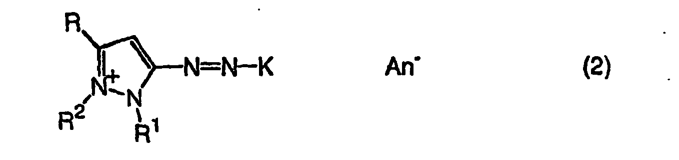 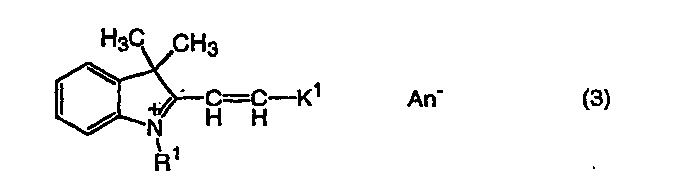 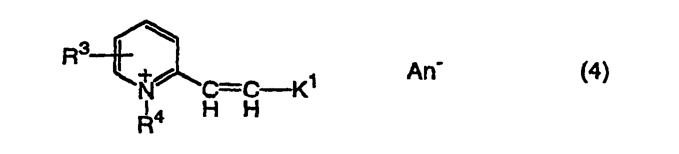 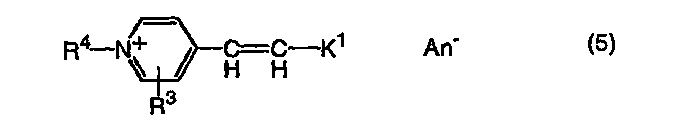 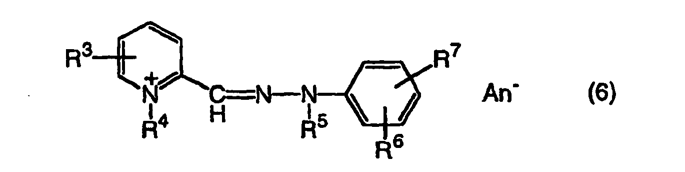 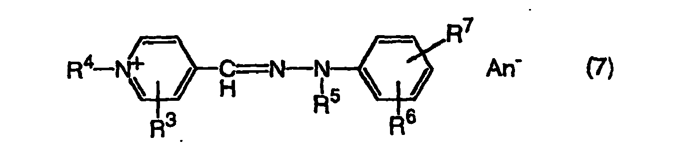 wherein, Y represents a methine group, a -CR²= group or a nitrogen atom,
R represents a hydrogen atom, a lower alkyl group, a chlorine atom or a nitro group,
R' represents a hydrogen atom, a lower alkyl group, a chlorine atom, a nitro group, a methylthio group, an amino group, a mono(lower alkyl)amino group or a di(lower alkyl)amino group,
R¹ and R² each independently represents a lower alkyl group which may have a substituent selected from a hydroxy group, lower alkoxy groups, halogen atoms, a cyano group, an amino group, mono(lower alkyl)amino groups and di(lower alkyl)amino groups,
R³ represents a hydrogen atom, a lower alkyl group or a cyano group,
R⁴ represents a lower alkyl group which may have a hydroxy or cyano group as a substituent,
R⁵ represents a hydrogen atom or a lower alkyl group, R⁶ and R⁷ each independently represents a hydrogen atom, a lower alkyl group, a lower alkoxy group or a halogen atom, or R⁵ and R⁶ form, together with the adjacent nitrogen atom and the carbon atom of the benzene ring, a 5- or 6-membered ring which may have a substituent,
K represents the residue of an aniline or phenol series, or heterocyclic coupling component,
K¹ represents the residue of an amino-substituted aromatic compound which may have a substituent or the residue of a nitrogen-containing heterocyclic compound, and
An⁻ represents an anion; and (B) a second direct dye selected from Basic Blue 7, Basic Blue 26, Basic Blue 47, , Basic Violet 1, Basic Violet 3, Basic Violet 4, Basic Violet 10, Basic Violet 14, Disperse Violet 1, Disperse Violet 15 and Disperse Violet 27.

### Best Modes for Carrying Out the Invention

In the formulas (1) to (7) representing the first direct dye to be used as the component (A), the term "lower" of the lower alkyl or lower alkoxy group means that the group referred to has 1 to 4 carbon atoms. Examples of such a lower alkyl group include methyl, ethyl, propyl, isopropyl, butyl, sec-butyl and tert-butyl groups, while those of the lower alkoxy group include methoxy, ethoxy, propoxy, isopropoxy, butoxy, isobutoxy and tert-butoxy groups. Examples of the halogen atom include fluorine, chlorine, and bromine atoms, with the chlorine atom being particularly preferred.

In the formula (1), preferred as R' are hydrogen atom, methyl group, ethyl group, amino group, mono- or dimethylamino group, and mono- or diethylamino group and preferred as Y is a methine group.

In the formula (2), preferred as R are a hydrogen atom and C₁₋₄ alkyl groups.

In the formulas (1), (2) and (3), preferred as R¹ are unsubstituted C₁₋₄ alkyl groups, while preferred as K are the residues of a coupling component represented by any one of the following formulas (8) to (12), particularly (8) or (9). 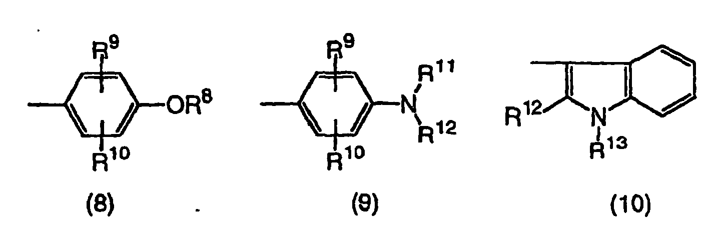 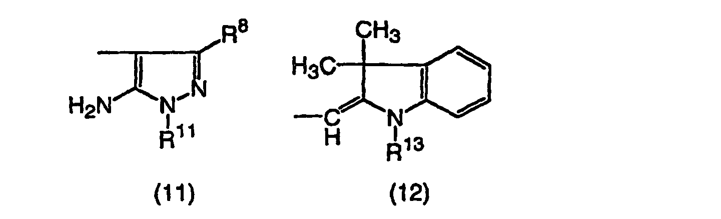 wherein, R⁸ represents a hydrogen atom or a lower alkyl group which may have a substituent selected from a hydroxy group, lower alkoxy groups, halogen atoms, a cyano group, an amino group, mono(lower alkyl)amino groups and di(lower alkyl)amino groups,

R⁹ and R¹⁰ each independently represents a hydrogen atom, a lower alkyl group, a lower alkoxy group or a halogen atom, R¹¹ and R¹² each independently represents a hydrogen atom or a lower alkyl group which may have a substituent selected from a hydroxy group, lower alkoxy groups, halogen atoms, a cyano group, an amino group, mono(lower alkyl)amino groups and di(lower alkyl)amino groups, or R¹¹ and R¹² form, together with the adjacent nitrogen atom, a 5- or 6-membered ring which may have a substituent, or R⁹ and R¹¹ or R¹⁰ and R¹² form, together with the adjacent nitrogen atom and the carbon atom of the benzene ring, a 5- or 6-membered ring which may have a substituent,

R¹³ represents a hydrogen atom or a lower alkyl group which may have a substituent selected from a hydroxy group, lower alkoxy groups, halogen atoms, a cyano group, an amino group, mono(lower alkyl)amino groups and di(lower alkyl)amino groups.

As R⁸, hydrogen atom and unsubstituted lower alkyl groups, particularly methyl and ethyl groups are preferred.

With regards to R⁹ to R¹², as each of R⁹ and R¹⁰, a hydrogen atom, lower alkyl groups, lower alkoxy groups and halogen atoms, particularly, a hydrogen atom, methyl, ethyl, methoxy and ethoxy groups and a chlorine atom are preferred, while as each of R¹¹ and R¹², a hydrogen atom and unsubstituted lower alkyl groups, particularly a hydrogen atom and methyl and ethyl groups are preferred. In the formula (9), it is also preferred that two of these groups are joined to form a ring as described below.

Described specifically, when R¹¹ and R¹² form, together with the adjacent nitrogen atom, a 5- or 6-membered ring which may have a substituent, the ring may be substituted with a lower alkyl or lower alkoxy group, but unsubstituted ring is preferred. Examples of the above-described 5- or 6-membered ring include pyrrolidine, piperidine, morpholine and piperazine rings. When R⁹ and R¹¹ or R¹⁰ and R¹² form, together with the adjacent nitrogen atom and the carbon atom of the benzene ring, a 5- or 6-memberted ring which may have a substituent, the ring may further have a hetero atom such as oxygen or sulfur. Alternatively, the ring may be substituted with a hydroxy group, alkoxy group, alkyl group, halogen atom or cyano group or may carry a further fused benzene ring. Preferred examples of the 5- or 6-membered ring include pyrrolidine, piperidine, morpholine and piperazine rings.

As R¹³, hydrogen atom and unsubstituted lower alkyl groups, particularly hydrogen atom and methyl and ethyl groups are preferred.

In the formulas (3), (4) and (5), groups represented by any one of the following formulas (13) to (15) are preferred as K¹. 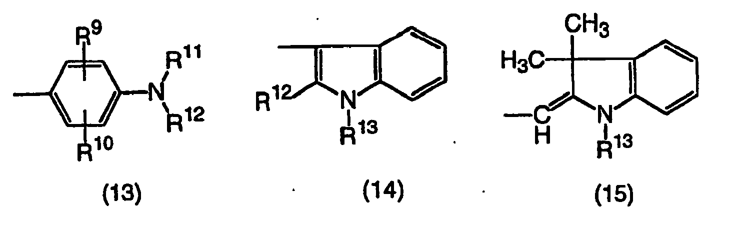 in the formulas, R⁹ to R¹³ each has the same meaning as described above.

As R⁹ to R¹³ representing K¹, similar groups to K are preferred.

In the formulas (6) and (7), preferred as R⁵ are hydrogen atom and methyl group, while preferred as each of R⁶ and R⁷ are hydrogen atom and methyl, ethyl, methoxy and ethoxy groups. Alternatively, it is preferred that R⁵ and R⁶ form a ring as described below.

Described specifically, when R⁵ and R⁶ form, together with the adjacent nitrogen atom and the carbon atom of the benzene ring, a 5- or 6-membered ring which may have a substituent, the ring may further have a hetero atom such as oxygen or sulfur atom. The ring may be substituted with a hydroxy group, an alkoxy group, an alkyl group, a halogen atom, a cyano group or a phenyl group or carry a further fused benzene ring. Preferred examples of the above-described 5- or 6-membered ring include pyrrolidine, piperidine, morpholine and piperazine rings.

In the direct dye to be used as the component (A), examples of the anion represented by An⁻ in the formulas (1) to (7) include chloride, bromide, iodide, trichlorozincic acid, tetrachlorozincic acid, sulfuric acid, hydrosulfuric acid, methyl sulfate, phosphoric acid, formic acid and acetic acid ions.

The following are specific examples of the direct dye to be used as the component (A).

Particularly preferred eight compounds:

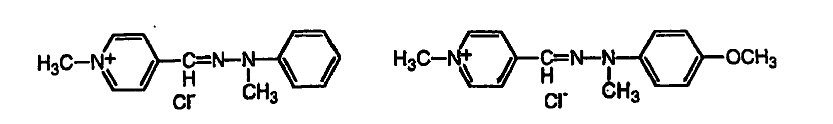

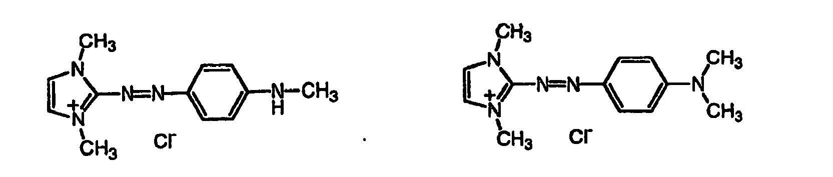

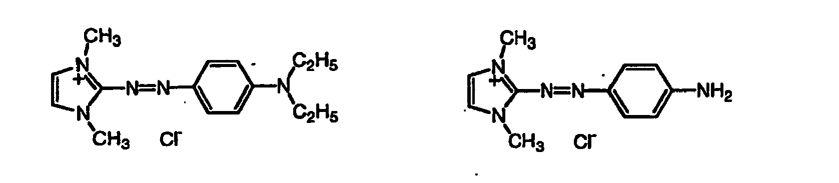

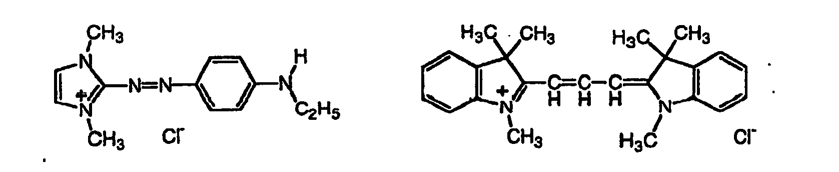

Further specific examples:

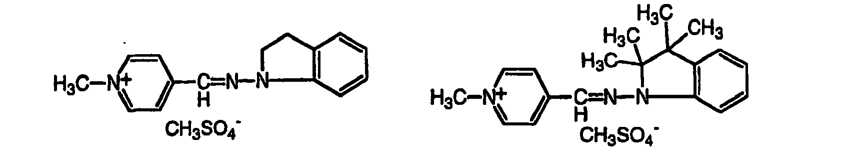

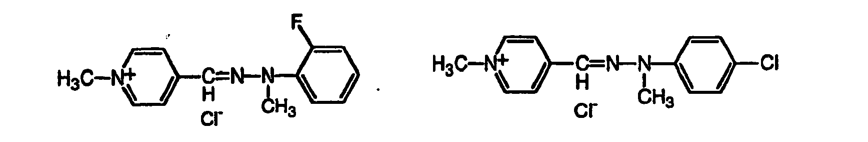

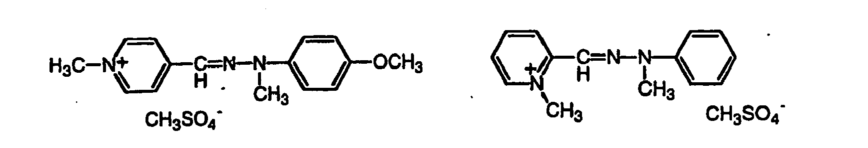

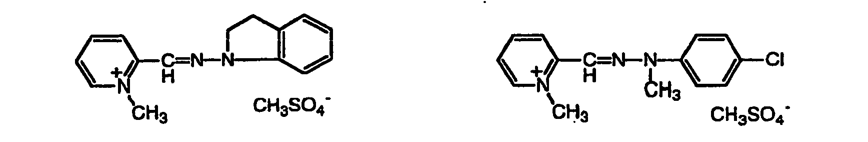

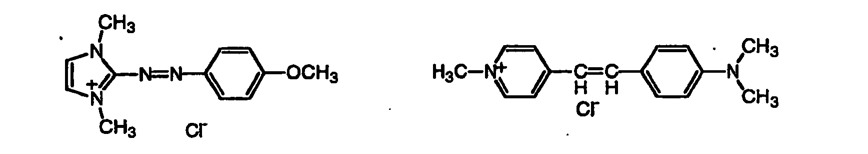

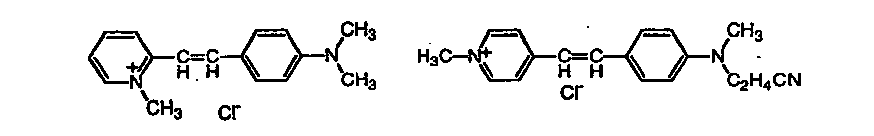

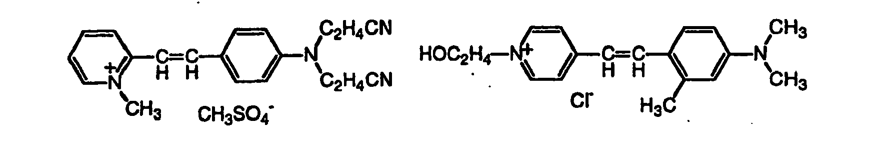

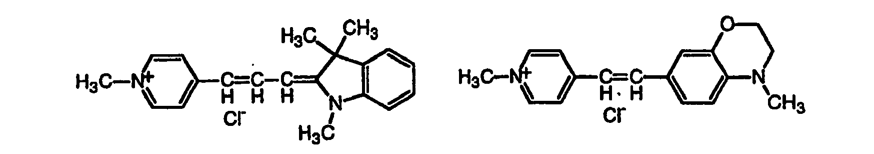

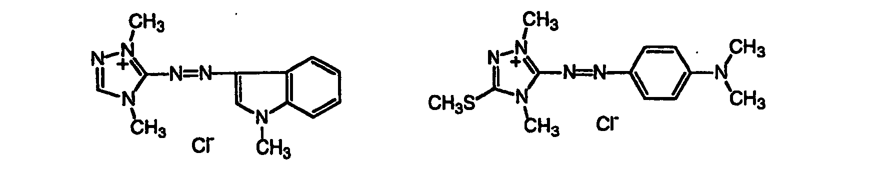

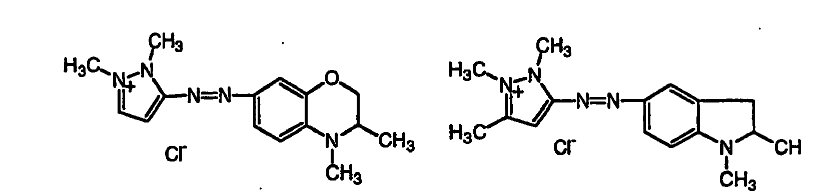

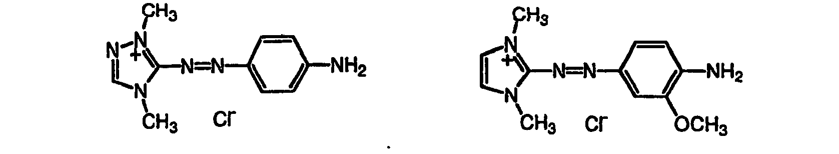

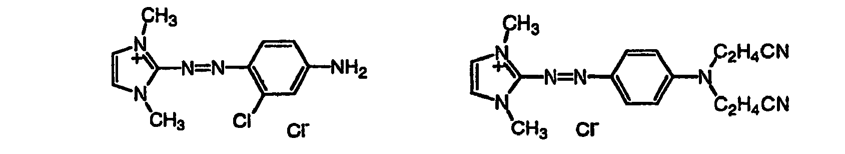

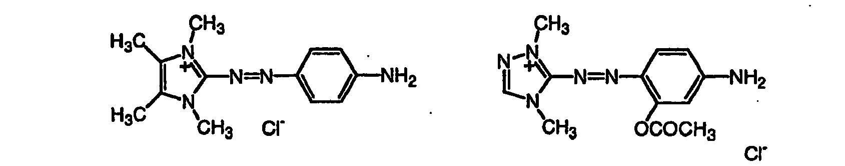

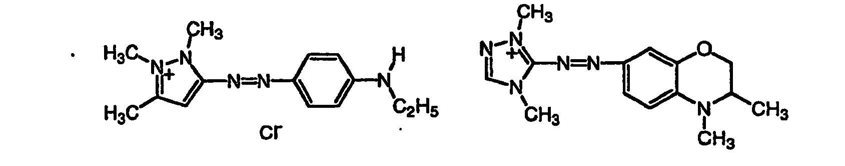

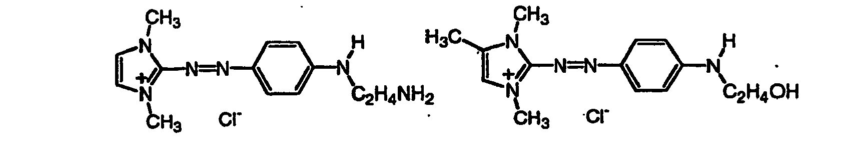

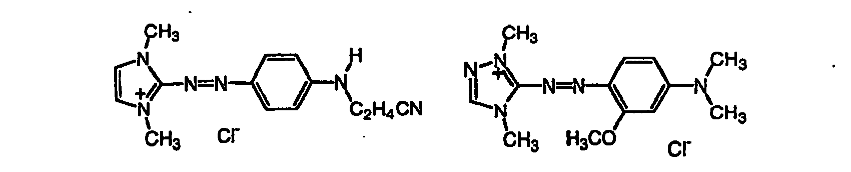

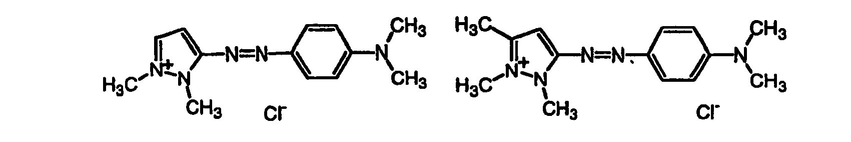

Among the above-described specific examples of the direct dye as the component (A), the first eight compounds are particularly preferred. As the component (A), the direct dyes (1) to (7) can be used either singly or in combination and its (or their) content is preferably 0.001 to 10 wt.%, with 0.01 to 5 wt.% being particularly preferred, each based on the whole composition.

As the second dye to be used as the component (B), Basic Blue 7 and Basic Blue 26 are particularly preferred. As the component (B), the direct dyes may be used either singly or in combination and its (or their) content is preferably 0.001 to 10 wt.%, with 0.01 to 5 wt.% being particularly preferred, each based on the whole composition.

In addition to the above-described components, 10 to 99 wt.% of water as a dissolution assistant for dye, a pH regulator to be added in an amount to regulate the pH of the hair dye composition within the below-described desired range and other additives may be incorporated as needed in the hair dye composition of the present invention.

The hair dye composition of the present invention can be formed into any one of liquid, emulsion, cream, gel, paste or mousse in a conventional manner. It can also be formed into an aerosol.

The pH of the hair dye composition of the present invention falls within a range of 8 to 12, particularly 8.5 to 11 upon usage. The pH is adjusted to 8 or greater in order to attain sufficient dyeing power and to 12 or less in order to avoid excessive irritation. Use of a volatile alkali such as ammonia is preferred for removing an alkali content completely, while use of an alkanolamine such as monoethanolamine is preferred for suppressing an offensive odor. Use of an acid in combination to form a buffer system heightens dyeing power and is therefore preferred.

According to the hair dyeing method of the present invention, the hair dye composition of the present invention is applied directly to the hair upon usage.

### -Examples-

### Examples 1, 2 and Comparative Examples 1, 2

In Table 1, shown are the composition of each of the hair dyes; and the color of the hair dyed by the below-described method with each of the hair dyes immediately after they were prepared or stored at 25°C for 30 days.

### (Dyeing method)

To a hair bundle of a white goat, the same weight of each of the hair dyes was applied uniformly. After it was allowed to stand for 10 minutes in a thermostat set at 30°C, the dyed hair bundle was rinsed with water of 40°C and then dried.

**Table 1**

| (wt.%) | Example | | Comp. Ex. | |
|---|---|---|---|---|
| | 1 | 2 | 1 | 2 |
| (A): Dye A (red) | - | 0.1 | - | 0.1 |
| (A): Dye B (orange) | 0.15 | 0.15 | 0.15 | 0.15 |
| (A): Dye C (yellow) | 0.15 | - | 0.15 | - |
| (B): Basic Blue 7 | 0.1 | - | - | - |
| (B): Basic Blue 26 | - | 0.25 | - | - |
| Dye D (blue) | - | - | 0.1 | - |
| Dye E (blue) | - | - | - | 0.25 |
| Monoethaholamine | 0.1 | | | |
| Phosphoric acid | Amount to adjust pH to 8.5 to 9 | | | |
| Perfume | q.s. | | | |
| Water | Balance | | | |
| Color shade of the dyed hair (with hair dye rightly after preparation) | Brown | Black | Brown | Black |
| Color shade of dyed hair (with hair dye after storage at 25°C for 30 days) | Brown | Black | Orange | Red |

### Dye A (red)

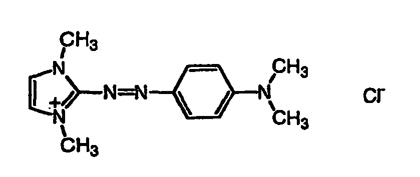

Dye prepared in Example 4 of Japanese Language Laid-Open Publication (PCT) No. Hei 8-501322

### Dye B (orange)

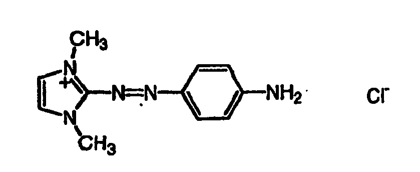

Dye prepared in Example 46 of Japanese Language Laid-Open Publication (PCT) No. Hei 8-501322

### Dye C (yellow)

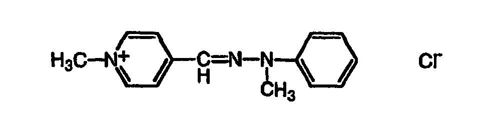

Dye prepared in Example 1 of Japanese Language Laid-Open Publication (PCT) No. Hei 8-501322

### Dye D (blue)

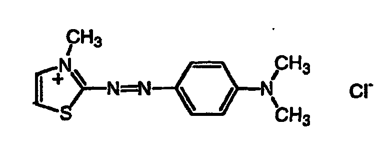

Dye prepared in Example 39 of Japanese Language Laid-Open Publication (PCT) No. Hei 8-501322

### Dye E (blue)

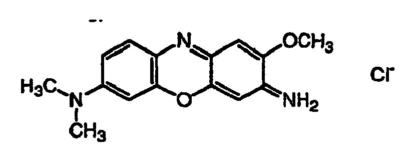

Dye prepared in Example 6 of Japanese Language Laid-Open Publication (PCT) No. Hei 8-501322

### Examples 3 to 7

In Table 2, shown are the composition of each of the hair dyes; and the color of the hair dyed, by a similar method to Example 1, with each of the hair dyes which was stored at 25°C for 30 days.

**Table 2**

| (wt.%) | Example | | | | |
|---|---|---|---|---|---|
| | 3 | 4 | 5 | 6 | 7 |
| (A): Dye A (red) | 0.1 | - | 0.1 | 0.1 | 0.1 |
| (A): Dye B (orange) | - | 0.15 | 0.2 | 0.15 | 0.2 |
| (A): Dye C (yellow) | 0.3 | 0.15 | - | - | - |
| (B): Basic Blue 7 | 0.1 | - | 0.1 | 0.25 | 0.1 |
| (B): Basic Blue 26 | - | 0.1 | - | - | 0.1 |
| Ethanol | - | 5 | - | 5 | 5 |
| Propylene glycol | - | - | 5 | - | 5 |
| Diethylene glycol monoethyl ether | - | 10 | - | - | - |
| Guar gum | 1 | - | - | - | - |
| Hydroxypropyl guar gum | - | 1 | 1 | 1 | 1 |
| "Merquat 100°, (product of Calgon Corp., a 40 wt.% solution) | - | 4 | 4 | - | - |
| "Merquat 280", (product of Calgon Corp., a 35 wt.% solution) | 3 | - | - | - | 1 |
| "Polyether-modified Silicone KF6005" (product of Shin-etsu Chemical) | - | - | - | - | 0.3 |
| "Amodimethicone SM8702C° (product of Dow Corning Toray Silicone, a 40 wt.% emulsion) | - | - | - | 1.5 | 1 |
| Monoethanolamine | 0.1 | | | | |
| Phosphoric acid | Amount to adjust pH 8.5 to 9 | | | | |
| Perfume | q.s. | | | | |
| Water | Balance | | | | |
| Color shade of the dyed hair (with a hair dye stored at 25°C for 30 days) | Brown | Light brown | Purplish brown | Black | Black |

## Claims

1. A hair dye composition which has a pH of 8 to 12 and comprises (A) a first direct dye represented by any one of the following formulas (1) to (7): 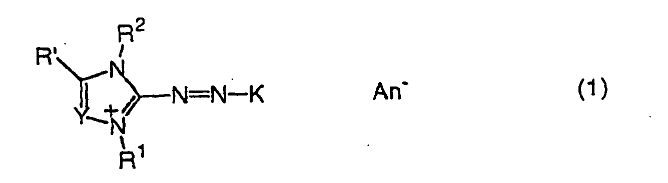 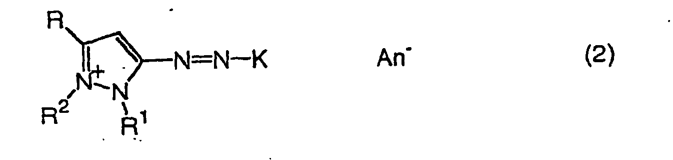 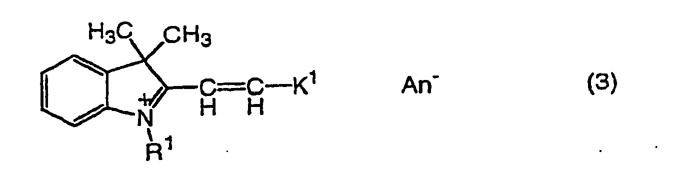 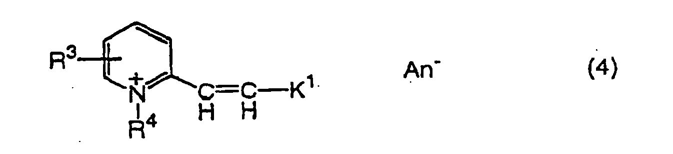 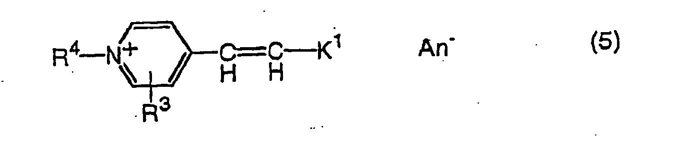 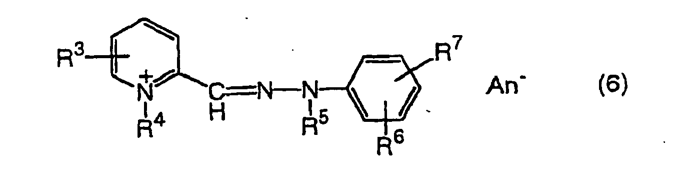 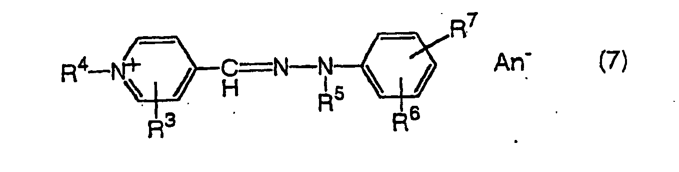 wherein, Y represents a methine group, a -CR²= group or a nitrogen atom,
R represents a hydrogen atom, a C₁₋₄ alkyl group, a chlorine atom or a nitro group,
R' represents a hydrogen atom, a C₁₋₄ alkyl group, a chlorine atom, a nitro group, a methylthio group, an amino group, a mono( C₁₋₄ alkyl)amino group or a di(C₁₋₄ alkyl)amino group,
R¹ and R² each independently represents a C₁₋₄ alkyl group which may have a substituent selected from a hydroxy group, C₁₋₄ alkoxy groups, halogen atoms, a cyano group, an amino group, mono( C₁₋₄ alkyl)amino groups and di(C₁₋₄ alkyl)amino groups,
R³ represents a hydrogen atom, a C₁₋₄ alkyl group or a cyano group,
R⁴ represents a C₁₋₄ alkyl group which may have a hydroxy or cyano group as a substituent,
R⁵ represents a hydrogen atom or a C₁₋₄ alkyl group, R⁶ and R⁷ each independently represents a hydrogen atom, a C₁₋₄ alkyl group, a C₁₋₄ alkoxy group or a halogen atom, or R⁵ and R⁶ form, together with the adjacent nitrogen atom and the carbon atom of the benzene ring, a 5- or 6-membered ring which may have a substituent,
K represents the residue of an aniline or phenol series or heterocyclic coupling component,
K¹ represents the residue of an amino-substituted aromatic compound which may have a substituent or the residue of a nitrogen-containing heterocyclic compound, and
An⁻ represents an anion; and (B) a second direct dye
selected from Basic Blue 7, Basic Blue 26, Basic Blue 47,
Basic Violet 1, Basic Violet 3, Basic Violet 4, Basic Violet 10, Basic Violet 14, Disperse Violet 1, Disperse Violet 15 and Disperse Violet 27.

2. A hair dyeing method, which comprises applying a hair dye composition as claimed in claim 1 to the hair.

## Patentansprüche

1. Haarfärbezusammensetzung, die eine pH von 8 bis 12 hat und (A) einen ersten direkziehenden Farbstoff mit einer der folgenden Formeln (1) bis (7) enthält: 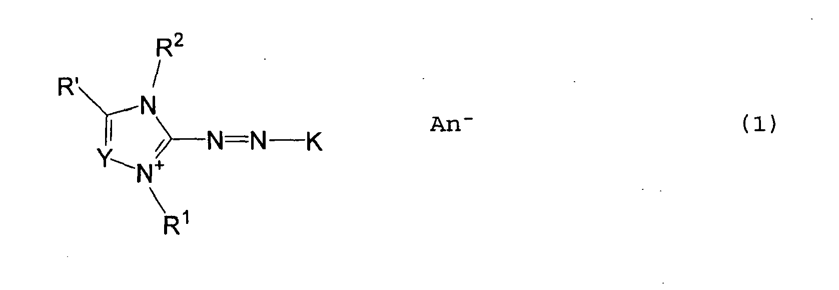 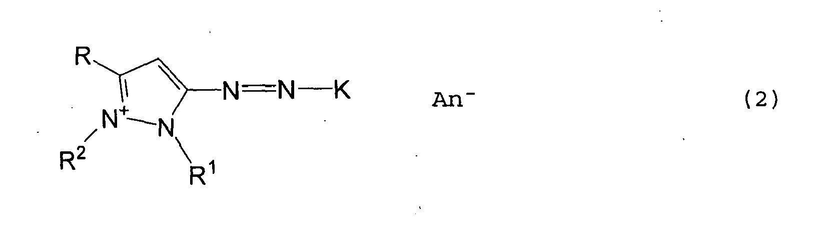 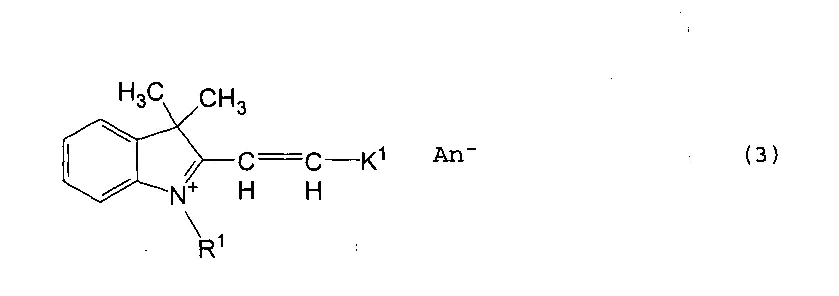 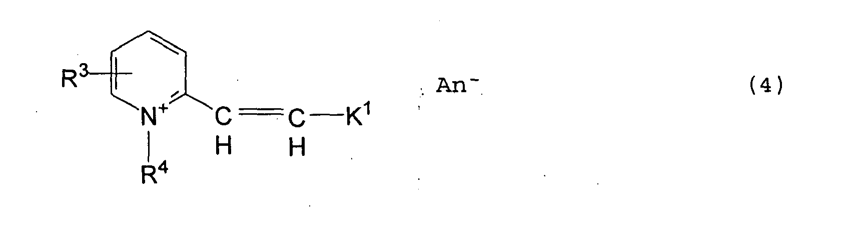 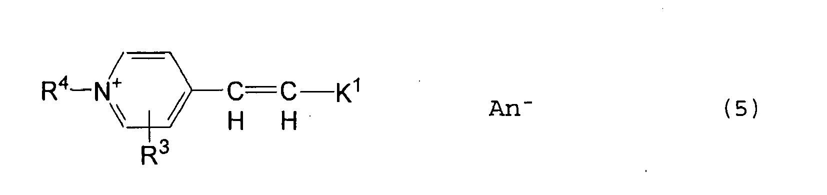 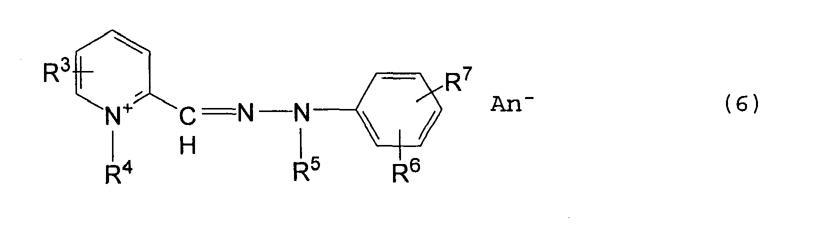 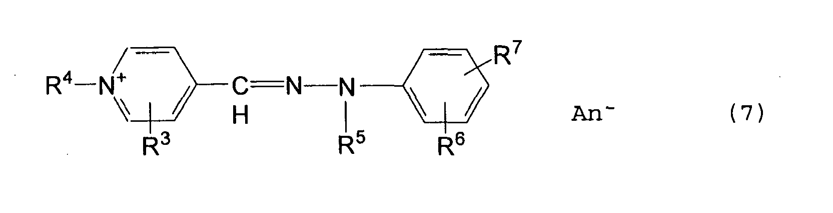 worin Y eine Methingruppe, eine -CR²= Gruppe oder ein Stickstoffatom ist,
R ein Wasserstoffatom, C₁₋₄-Alkylgruppe, Chloratom oder Nitrogruppe ist,
R' ein Wasserstoffatom, C₁₋₄-Alkylgruppe, Chloratom, Nitrogruppe, Methylthiogruppe, Aminogruppe, Mono(C₁₋₄-alkyl)aminogruppe oder Di(C₁₋₄-alkyl)aminogruppe ist,
R¹ und R² jeweils unabhängig eine C₁₋₄-Alkylgruppe sind, die einen Substituenten haben kann, ausgewählt aus einer Hydroxygruppe, C₁₋₄-Alkoxygruppe, Halogenatomen, Cyanogruppe, Aminogruppe, Mono(C₁₋₄-alkyl)aminogruppen und Di(C₁₋₄-alkyl)aminogruppen
R³ ein Wasserstoffatom, eine C₁₋₄-Alkylgruppe oder Cyanogruppe ist,
R⁴ eine C₁₋₄-Alkylgruppe ist, die eine Hydroxy- oder Cyanogruppe als Substituenten haben kann,
R⁵ ein Wasserstoffatom oder eine C₁₋₄-Alkylgruppe ist, R⁶ und R⁷ jeweils unabhängig ein Wasserstoffatom, eine C₁₋₄-Alyklgruppe, C₁₋₄-Alkoxygruppe oder Halogenatom sind oder R⁵ und R⁶ zusammen mit dem benachbarten Stickstoffatom und dem Kohlenstoffatom des Benzolrings einen 5- oder 6-gliedrigen Ring bilden können, der einen Substituenten haben kann,
K den Rest einer Anilin- oder Phenolserie oder heterozyklischen Kupplungskomponente bedeutet,
K¹ der Rest einer aminosubstituierten aromatischen Verbindung, die einen Substituenten haben kann, oder der Rest einer stickstoffhaltigen heterozyklischen Verbindung ist und An- ein Anion ist,
und (B) einen zweiten direkziehenden Farbstoff ausgewählt aus Basic Blue 7, Basic Blue 26, Basic Blue 47, Basic Violet 1, Basic Violet 3, Basic Violet 4,
Basic Violet 10, Basic Violet 14, Disperse Violet 1, Disperse Violet 15 und Disperse Violet 27 enthält.

2. Haarfärbeverfahren, umfassend das Auftragen einer Haarfärbezusammensetzung wie in Anspruch 1 definiert, auf das Haar.

## Revendications

1. Composition de colorant capillaire qui a un pH de 8 à 12 et qui comprend (A) un premier colorant direct représenté par l'une quelconque des formules suivantes (1) à (7): 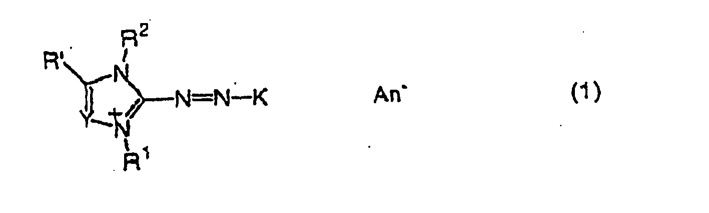 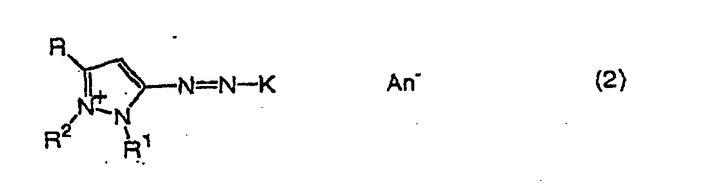 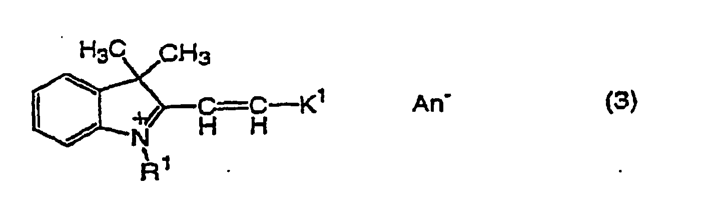 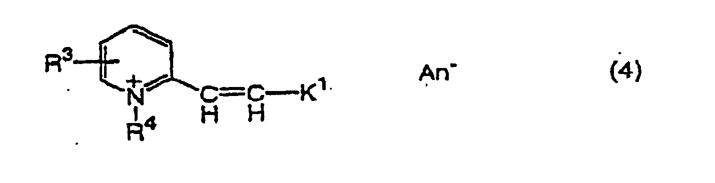 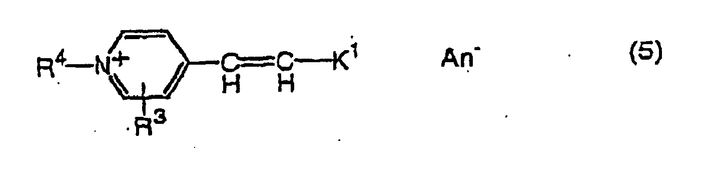 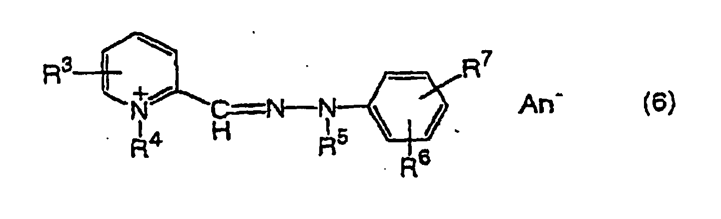 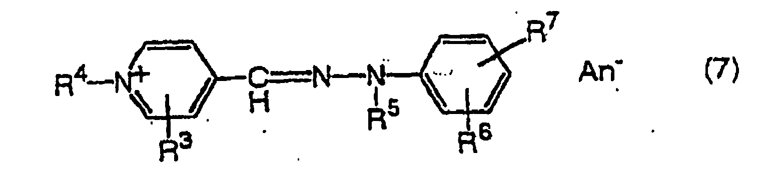 Où, Y représente un groupe méthine, a groupe -CR²= ou un atome d'azote,
R représente un atome d'hydrogène, un groupe alkyle C₁₋₄, an atome de chlore ou un groupe nitro,
R' représente un atome d'hydrogène, un groupe alkyle C₁₋₄, un atome de chlore, un groupe nitro, un groupe méthylthio, un groupe amino, un groupe monoalkyle C₁₋₄ amino ou un groupe dialkyle C₁₋₄ amino,
R¹ et R² chacun représente indépendamment un groupe alkyle C₁₋₄ qui peut avoir un substituant sélectionné parmi un groupe hydroxy, des groupes alkoxy C₁₋₄, des atomes d'halogène, un groupe cyano, un groupe amino, des groupes monoalkyle C₁₋₄ amino, et des groupes dialkyle C₁₋₄ amino,
R³ représente un atome d'hydrogène, un groupe alkyle C₁₋₄ ou un groupe cyano,
R⁴ représente un groupe alkyle C₁₋₄ qui peut avoir un groupe hydroxy ou un groupe cyano comme substituant.
R⁵ représente un atome d'hydrogène ou un groupe alkyle C₁₋₄, R⁶ et R⁷ chacun représente indépendamment un atome d'hydrogène, un groupe alkyle C₁₋₄, un groupe alcoxy C_{1- 4} ou un atome d'halogène, ou R⁵ et R⁶ forment, conjointement avec l'atome d'azote adjacent et l'atome de carbone du cycle benzénique, un cycle à cinq ou à six éléments qui peut avoir un substituant,
K représente le résidu d'une série aniline ou phénol ou de composant de couplage hétérocyclique,
K' représente le résidu d'un composé aromatique amino-substitué qui peut avoir un substituant ou le résidu d'un composé hétérocyclique contenant de l'azote, et
An⁻ représente un anion;
et (B) un deuxième colorant direct sélectionné parmi le Basic Blue 7, Basic Blue 26, Basic Blue 47, Basic Violet 1, Basic Violet 3, Basic Violet 4, Basic Violet 10, Basic Violet 14, Disperse Violet 1, Disperse Violet 15 et le Disperse Violet 27.

2. Procédé de coloration capillaire, qui comprend l'application d'une composition de colorant capillaire comme revendiquée dans la revendication 1.
